(19) Europäisches Patentamt
European Patent Office
Office européen des brevets

(11) Publication number: **0 165 458 A2**

(12) # EUROPEAN PATENT APPLICATION

(21) Application number: 85105872.7

(22) Date of filing: 13.05.85

(51) Int. Cl.[4]: C 07 D 307/92
A 61 K 7/46, A 23 L 1/226

(30) Priority: 14.05.84 JP 94638/84

(43) Date of publication of application: 27.12.85 Bulletin 85/52

(84) Designated Contracting States: CH DE FR GB LI NL

(71) Applicant: T. HASEGAWA COMPANY, LTD.
No. 9, Nihonbashi-Honcho 4-chome
Chuo-ku Tokyo(JP)

(72) Inventor: Kawanobe, Tsuneo
1-12-805, Wakabadai Asahi-ku
Yokohama-shi Kanagawa-ken(JP)

(72) Inventor: Kogami, Kunio
655-11, Nougaya
Machida-shi Tokyo(JP)

(74) Representative: Kraus, Walter, Dr. et al,
Patentanwälte Kraus Weisert & Partner
Thomas-Wimmer-Ring 15
D-8000 München 22(DE)

(54) **(+)-Ambrox, process for production thereof and use thereof.**

(57) (±)−3a,6,6,9a-Tetramethyldodecahydronaphtho-(2,1,b)furan of the following formula (A):

(±) 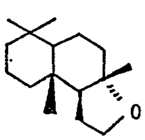 ..... (A)

a process for producing the formula (A) compound and a perfuming or flavoring composition containing the formula (A) compound.

EP 0 165 458 A2

Croydon Printing Company Ltd.

This invention relates to (±)-3a,6,6,9a-tetramethyldodecahydronaphtho-(2,1,b)furan [to be referred to hereinbelow as (±)-ambrox] of the following formula (A)

(±) 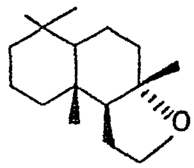 ..... (A)

a process for producing the compound of formula (A), and the use of the compound of formula (A) as a perfuming or flavoring modifier.

Ambergris has previously been known as a material useful in the field of perfumes or flavors. It is a very important and expensive animal perfume material obtained by drying a foreign material formed in the intestinal tract of the sperm whale, which is considered as a calculus. Usually, ambergris is dissolved in alcohol to form a tincture and used as one component of various perfuming or flavoring properties. In recent years, however, it has become difficult to procure ambergris largely because of the restrictions of whale catching.

The presence of various perfuming and flavoring components in the tincture of ambergriss is known. The key substance of the perfuming components is (-)-ambrox represented by the following formula (B).

(-) 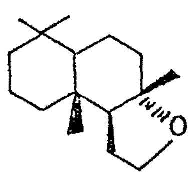 ..... (B)

The (-)-ambrox of formula (B) has four asymmetric carbon atoms, and therefore, eight stereoisomers possibly exist. But the optically active (-)-isomer shown in formula

(B) in which the fused ring-forming moiety shows a trans steric configuration is the only compound which occurs naturally, has high diffusibility and gives off a very strong typical ambergris odor.

Because of the difficulty of obtaining ambergris, chemically synthesized compounds of formula (B) have recently been used as a material for perfuming or flavoring preparations.

One typical prior method of synthesizing (-)-ambrox of formula (B) comprises oxidizing (-)-sclareol, an optically active compound present in the essential oil of naturally occurring <u>Salvia</u> <u>sclarea</u>, with chromic acid to synthesize (+)-norambreinolide [Helv. Chim. Acta., 14, 570 (1931)], then reducing the (+)-norambreinolide and cyclizing the reduction product as schematically shown below [Dragoco Report, 11/12, 276-283 (1979)].

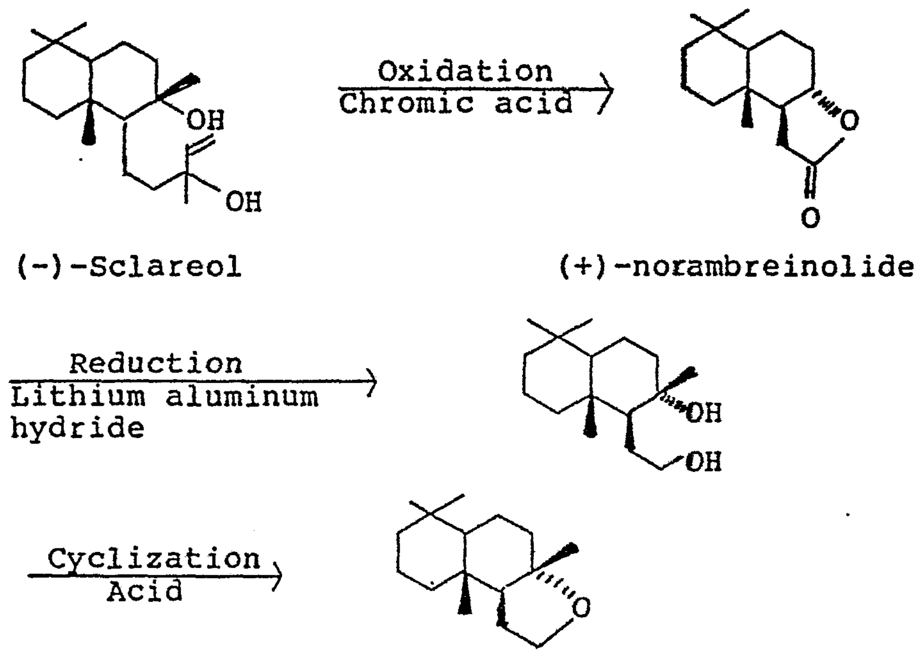


(-)-Sclareol

(+)-norambreinolide

(B) (-)-Ambrox

However, the above method has many defects and disadvantages. For example, the starting sclaerol is an expensive material present only in several percent in the essential oil of <u>Salvia</u> <u>sclarea</u>. Its supply is unstable

since it is derived from the naturally occurring material. The use of hazardous chromic acid as a reagent gives rise to an industrial hygienic problem. Moreover, the method uses aluminum lithium hydride which is highly inflammable and not suitable for industrial use. The (-)-ambrox produced is very expensive and gives rise to an economical problem in using it as a material for perfuming compositions.

Chemistry Letters, pp. 757-760 (1981 and pp. 729-732 (1983) proposed a method schematically shown below of synthesizing (±)-norambreinolide of formula (2) below, an intermediate of the compound of formula (A) of this invention, which comprises reacting (±)-trans-β-monocyclohomofarnesylic acid in dichloromethane in the presence of tin tetrachloride, and discussed the reactivity of the above farnesylic acid in the case of using other Lewis acids ($SnBr_4$, $TiCl_4$, $BF_3$-$Et_2O$) or protonic acids (sulfuric acid, trichloroacetic acid).

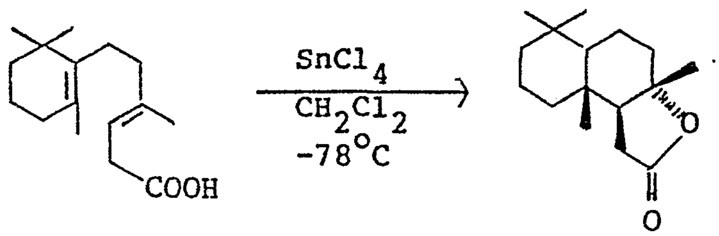


(2) (Yield 57%)

This method, however, requires an extremely low temperature of $-78^oC$ which is not suitable for industrial practice. Furthermore, isomers are formed besides the compound of formula (2), and the yield of the compound of formula (2) is as low as about 57% which is not entirely satisfactory. The above literature references state that with the above catalysts other than tin tetrachloride, no marked effect of stereoselectivity is obtained.

The optically active (-)-ambrox of formula (B) is very expensive, and cannot be widely and economically used as a material for various perfuming or flavoring compositions.

The present inventors have made extensive investigations in order to develop a process for synthesizing optically inactive (±)-ambrox of formula (A) in which the fused ring forming moiety shows a trans steric configuration (natural type) and which has not been synthesized heretofore, by a total chemical synthesis without using a naturally occurring material, and to elucidate the perfume characteristics of the compound of fromula (A).

These investigations have led to the discovery that optically inactive (±)-ambrox of formula (A) in which the fused ring forming moiety has a trans steric configuration can be produced industrially advantageously at low cost from an inexpensive raw material which is easy to synthesize or obtain. In particular, the present inventors have found that optically inactive (±)-ambrox of formula (A) can be totally synthesized industrially easily at low cost by cyclizing (±)-2,5,5,8a-tetramethyl-1-(2-hydroxyethyl)-2-hydroxydecalin of the following formula (3)

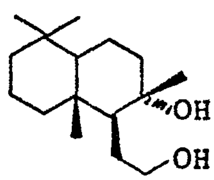

..... (3)

in the presence of a base and p-toluenesulfonyl chloride. The compound of formula (3) can be easily synthesized in two steps from a known compound, (±)-trans-β-monocyclohomofarnesylic acid or its ester of the following formula (1)

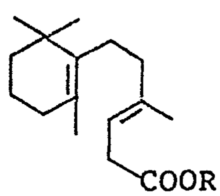

..... (1)

wherein R represents a hydrogen atom or a methyl or ethyl group,

which in turn can be easily synthesized from inexpensive dihydro-β-ionone which is easy to synthesize or obtain.

It has been unexpectedly found that the optically inactive compound of formula (A) has an amber-like fragrance comparable to the fragrance of the optically active (-)-ambrox of formula (B) synthesized from sclareol with an excellent lasting effect. Investigations of the present inventors have shown that the compound of formula (A) can be synthesized industrially advantageously with more ease and at lower costs than the optically active (-)-ambrox of formula (B), and can be used widely at low cost as a material for various perfuming and flavoring compositions.

It has been found that the compound of formula (A) well harmonizes with various natural and synthetic perfumes, and can be used as a soft, mild and long-lasting perfuming or flavoring modifier for various perfume compositions of woody, floral, green, fruity, musky and citrus notes.

It is an object of this invention therefore to provide the composition of formula (A) not described in the prior literature, a process for producing the compound (A), and a perfuming or flavoring composition comprising as an active ingredient the compound of formula (A) which has never been used heretofore.

The above and other objects and advantages will become more apparent from the following description.

The present invention provides (±)-3a,6,6,9a-tetramethyldodecahydronaphtho(2,1,b)furan of the following formula (A):

(±) 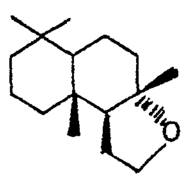 ..... (A)

This compound can be synthesized by a process which comprises

(i) reacting a (±)-trans-β-monocyclohomofarnesylic acid or an ester thereof represented by the following formula (1)

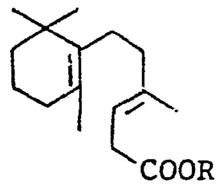

..... (1)

wherein R represents a hydrogen atom or a methyl or ethyl group,

with trifluoroacetic acid to form decahydro-3a,6,6,9a-tetramethyl-(3aα,5aβ,9aα,9bβ)-(±)-naphtho[2,1-b]furan-2-(1H)one of the following formula (2)

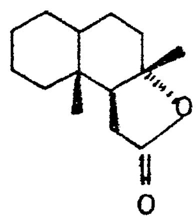

..... (2)

(ii) reducing the compound of formula (2) with bis-(2-methoxyethoxy)-aluminum sodium hydride [$NaAlH_2(OCH_2CH_2OCH_3)_2$] to form (±)-2,5,5,8a-tetramethyl-1-(2-hydroxyethyl)-2-hydroxydecalin of the following formula (3)

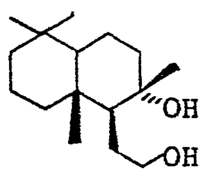

..... (3)

and

(iii) cyclizing the compound of formula (3) by contacting it with p-toluenesulfonyl chloride in the presence of a base to form the compound of formula (A).

The above process for producing the optically inactive (±)-ambrox of formula (A) can be shown by the following scheme. The compound of formula (A) can be synthesized industrially advantageously at low cost.

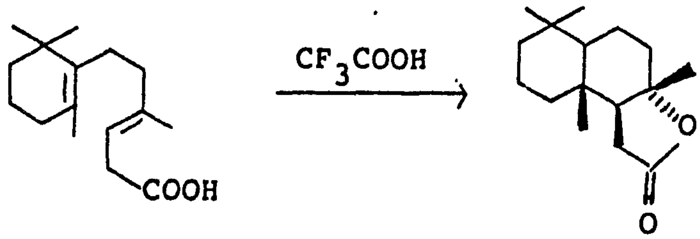


(1) (±)-trans-β-monocyclohomofarnesylic acid

(2) (±)-norambrenolide

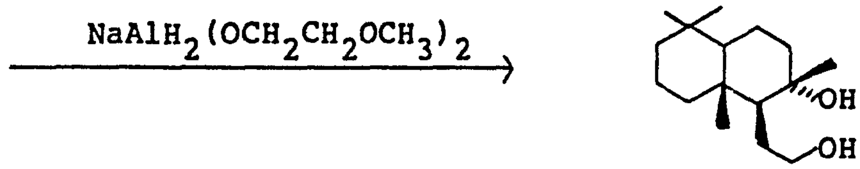


(3)

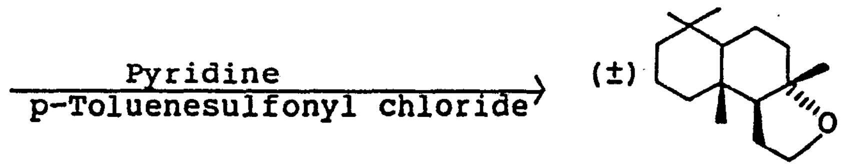


(A)

In the above process, the starting (±)-trans-β-monocyclohomofarnesylic acid of formula (1) can be easily synthesized from, for example, dihydro-β-ionone. By converting the resulting cis- and trans- isomeric mixture into an ethyl ester and subjecting it to precise distillation, the compound of formula (1) can be easily obtained.

The (±)-norambrenolide can be easily synthesized, for example, by catalytically reacting (±)-trans-β-monocyclohomofarnesylic acid which can be obtained as above with, for example, trifluoroacetic acid, optionally in an organic solvent. The reaction can be easily carried out, for example, at a temperature of about -20$^{o}$C to about 50$^{o}$C for about 10 minutes to about 5 hours. Trifluoroacetic acid used in the reaction is easily available on the market. The amount of trifluoroacetic acid is, for example, about 0.5 to about 10 times the weight of the compound of formula (1). Preferably, the organic solvent which may be used

is, for example, dichloromethane, carbon tetrachloride, benzene, hexane or toluene. There is no particular restriction on the amount of the organic solvent used. It is, for example, about 0.5 to about 10 times the weight of the compound of formula (1). After the reaction, the reaction mixture is successively washed with water and an aqueous solution of sodium hydrogen carbonate in a customary manner. The organic layer is dried and the solvent is evaporated to give a crude oil. As required, this oil can be purified by such a purifying means as silica gel chromatography (ether/pentane/benzene=4/1/2).

The (±)-2,5,5,8a-tetramethyl-1-(2-hydroxyethyl)-2-hydroxydecalin can be easily synthesized by, for example, reducing the compound of formula (2) obtained in step (i) with bis-(2-methoxyethoxy)-aluminum sodium hydride [$NaAlH_2(OCH_2CH_2OCH_3)_2$], as required in an organic solvent. This reaction can be carried out at a temperature of about 20 to about 80$^o$C for a period of about 1 to about 10 hours. The amount of bis(2-methoxyethoxy)-aluminum sodium hydride used is, for example, about 0.5 to about 2 times the weight of the compound of formula (2). Examples of the organic solvent are toluene, benzene, hexane and ether. The amount of the organic solvent used is, for example, about 1 to about 20 times the weight of the compound of formula (2). After the reaction, the reaction mixture is sucessively washed with an aqueous solution of sodium hydroxide, water, an aqueous solution of acetic acid and an aqueous solution of sodium hydrogen carbonate in a customary manner, and then dried. The organic solvent is then evaporated to give the compound of formula (3) as white crystals.

The (±)-ambrox of formula (A) in accordance with this invention can be easily synthesized by, for example, catalytically reacting the compound of formula (3) obtained as above with p-toluenesulfonyl chloride in the presence of a base. Organic bases such as pyridine, quinoline and triethylamine may be used as the base. The amount of the

base is, for example, about 1 to about 20 times the weight of the compound of formula (3). The amount of p-toluenesulfonyl chloride used is preferably about 1 to about 10 moles per mole of the compound of formula (3). The reaction can be carried out, for example, at a temperature of about -20$^{o}$C to about 50$^{o}$C for a period of about 1 to about 12 hours. After the reaction, the reaction mixture is poured into water and extracted with toluene, for example. The extract is washed successively with hydrochloric acid, water and an aqueous solution of sodium hydrogen carbonate in a customary manner. The organic solvent is evaporated to give the compound of formula (A) as white crystals.

The (±)-ambrox of formula (A), in spite of being an optically inactive isomer, has an amber-like odor comparable to the odor of the natural-type optically active (-)-ambrox. When it is mixed with other perfumes, it shows a marked duration and is usesul as a durable flavoring or perfuming modifier.

Thus, according to this invention, there is provided a perfuming or flavoring composition comprising a conventional perfuming or flavoring agent or carrier and, as a perfuming or flavoring modifier thereof, a modifying amount of (±)-3a,6,6,9a-tetramethyldodecahydronaphtho-(2,1,b)furan of the following formula (A):

(±) 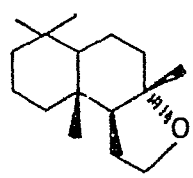 ...... (A)

The conventional perfuming or flavoring agent or carrier may be one or more of conventional synthetic or natural perfumes or flavors, and known carriers or diluents for perfumes or flavors.

The perfuming or flavoring composition of this invention imparts soft, mild and long-lasting fragrance or flavor as a perfuming or flavoring modifier for various

perfumes, foods and drinks, health-preserving and hygienic articles and medical drugs.

More specifically, when (±)-ambrox of formula (A) is blended with at least one synthetic perfume, a novel long-lasting perfuming or flavoring composition can be prepared.

When it is blended with reconstituted essential oils such as bergamot oil, lemon oil, geranium oil, mandarin oil or lavender oil, novel long-lasting perfuming or flavoring compositions having the mildness and body of natural essential oils can be prepared.

The (±)-ambrox of formula (A) also harmonizes well with various natural essential oils such as orange oil, lime oil, lemon oil, grapefruit oil, oak moss oil, citronella oil, vetiver oil, cinnamon oil, patchouli oil, thyme oil, clove oil, bergamot oil and rose oil, and novel long-lasting perfuming or flavoring compositions emphasizing the characteristics of the essential oils can be prepared.

Furthermore, when it is blended with blended perfumes comprising two or more natural perfumes and synthetic aromatic chemicals, for example fragrance compositions such as single floral perfumes such as rose, jasmin, lilac, osmanthus, carnation and lily of the valley, floral bouquets comprising combinations of these or modern floral bouquets or oriental bouqutes obtained by adding a green note, a spicy note or an aldehyde note to the aforesaid floral bouquets, or flavor compositions of strawberry, lemon, orange, grapefruit, apple or pineappple, there can be prepared perfuming or flavoring compositions which are mild, resemble natural perfumes or flavors and are long-lasting.

The amount of the (±)-ambrox of formula (A) to be blended is, for example, about 0.001 to about 50% by weight based on the weight of the composition.

Thus, according to the present invention, a

durable perfuming or flavoring fortifier composition comprising (±)-ambrox of formula (A) as an active ingredient. By utilizing this fortifier composition, there can be provided foods and drinks comprising the (±)-ambrox of formula (A) as a fragrant and flavoring component, perfumes and cosmetics comprising (±)-ambrox of formula (A) as a fragrant component, and health-preserving or hygienic articles, or medical drugs comprising (±)-ambrox of formula (A) as a fragrant or flavoring component. For example, (±)-ambrox of formula (A) can be incorporated in a suitable amount capable of imparting its unique fragrance or flavor into drinks such as fruit juices, wines or liquors produced by using fruits, milks and carbonated drinks; ice confectionary such as icecreams, sherbets and ice candies, other confectionary, jams, chewing gums, breads, coffee, cocoa and various types of tea, soups, flavors and seasonings, instant drinks and foods, and various snacks. The (±)-ambrox can also be incorporated in a suitable amount capable of imparing its unique fragrance into cosmetics such as skin care preparations, hair preparations, makeup preparations and bath products, detergents and soaps. It can also be incorporated into various health-preserving and hygienic materials used for health-preserving and hygienic purposes, toothpastes, tissue paper and toilet papers, and flavoring agents for facilitating administration of medical drugs.

The following examples further illustrate the production and use of (±)-ambrox of this invention.

EXAMPLE 1

Synthesis of decahydro3a,6,6,9a-tetramethyl-(3aα,5aβ,9aα,9bβ)-(±)-naphtho-[2,1-b]furan-2-(1H)-one [(±)-norambrenolide of formula (2)]:-

A mixture of 2.2 g of (±)-trans-β-monocyclohomofarnesylic acid and 2 ml of dichloromethane was added to 16 ml of trifluoroacetic acid at $0^{o}C$, and at this temperature, the mixture was stirred for 2 hours. The reaction mixture was poured into ice water and washed with an aqueous

solution of sodium hydrogen carbonate. The organic layer was dried over magnesium sulfate, and the solvent was evaporated. The resulting crude oil was chromatographed on a silica gel column using ether/pentane/benzene (4/1/2) as an eluent to give 1.4 g of (±)-norambrenolide in a yield of 24%.

Melting point: 119 - 121°C (hexane)

IR: 1770 $cm^{-1}$

$^1$H-NMR ($CDCl_3$)δ: 0.84 (3H, s), 0.89 (3H, s), 0.91 (3H, s), 1.34 (3H, s), 2.2-2.5 (2H, m).

EXAMPLE 2

Synthesis of (±)-2,5,5,8a-tetramethyl-2-(2-hydroxyethyl)-3-hydroxydecalin of formula (3):-

A mixture of 24 ml of dry toluene and 20 ml (8 millimoles) of a toluene solution of 70% bis-(2-methoxyethoxy)aluminum hydride was added to a solution composed of 1.2 g (5 millimoles) of (±)-norambrenolide and 12 g of dry toluene at 50°C over 1 hour. The mixture was then stirred for 2 hours at this temperature. After cooling, the reaction mixture was poured into 50 ml of a 10% aqueous solution of sodium hydroxide. The organic layer was successively washed with a 10% aqueous solution of sodium hydroxide, water, a 3% aqueous solution of acetic acid and an aqueous solution of sodium hydrogen carbonate. The organic solvent was evaporated to give 1.2 g of the diol of formula (3) in a yield of 99%.

EXAMPLE 3

Synthesis of (±)-3a,6,6,9a-tetramethyldodecahydronaphtho(2,1,b)furan [(±)-ambrox of formula (A)]:-

Three grams (16 millimoles) of p-toluenesulfonyl chloride was added to 1.2 g (5 millimoles) of the diol of formula (3) and 30 ml of dry pyridine at 0°C. The mixture was stirred for 2 hours at this temperature and then for 2 hours at room temperature (10 to 15°C), and then left to stand overnight.

The reaction mixture was poured into water, and

extracted with 200 ml of toluene. The extract was successively washed with 5% hydrochloric acid, water and an aqueous solution of sodium hydrogen carbonate. The solvent was evaporated to give 0.72 g of the compound of formula (A) as white crystals in a yield of 63%.

IR (KBr): 1000 $cm^{-1}$

$^1H$ NMR ($CDCl_3$)δ: 0.84 (6H, s), 0.88 (3H, s), 1.09 (3H, s), 3.85 (2H, m).

EXAMPLE 4

The following substances were blended as constituent components of a fragrance compound of a gardenia note.

| Component | Parts by weight |
|---|---|
| Benzyl acetate | 100 |
| Hydroxycitronellal | 150 |
| Coumarin | 10 |
| Jasmin absolute | 30 |
| Orange flower absolute | 20 |
| Phenylmethylcarbinyl acetate | 30 |
| Heliotropin | 100 |
| Bergamot oil | 100 |
| Linalool | 100 |
| Amylcinnamic aldehyde | 40 |
| Ylang-Ylang oil | 70 |
| Galaxolide | 30 |
| Alpha-ionone | 100 |
| Tolu-resinoide | 20 |
| Phenyl acetaldehyde | 15 |
| iso-Eugenyl acetate | 35 |
| Civet 10% ethyl alcohol | 10 |
| Octyl aldehyde 10% ethyl alcohol | 5 |
| Decyl aldehyde 10% ethyl alcohol | 5 |
| Cyclohexyl butyrate | 30 |

By adding 60 g of (±)-ambrox to 940 g of the above composition, a novel blended perfume composition of

a gardenia note which was natural and had a very good duration was obtained.

A blended perfume composition was prepared by adding 60 g of (-)-ambrox instead of (±)-ambrox to 940 g of the above composition.

The above compositions were compared by a panel of 10 specialists. Nine out of the 10 panelists reported that the blended perfume composition comprising the compound of this invention was more durable than the blended composition prepared for comparison.

EXAMPLE 5

The following substances were blended as constituent componnents of a blended perfume composition of a bouquet note.

| Components | Parts by weight |
|---|---|
| Phenyl ethyl alcohol | 180 |
| Linalyl acetate | 30 |
| Bergamot oil | 40 |
| Geranium oil | 50 |
| Benzyl acetate | 60 |
| Heliotropin | 80 |
| Geraniol | 110 |
| Lavender oil | 20 |
| β-ionone | 100 |
| Amyl salicylate | 45 |
| Terpinyl acetate | 135 |
| Ceder wood oil | 100 |
| Citronellol | 50 |
| | 1000 |

By adding 20 g of (±)-ambrox to 980 g of the above composition, a novel perfume composition of a bouquet note which was natural and had an excellent duration was prepared.

A blended perfume composition was prepared by

adding 20 g of (-)-ambrox instead of (±)-ambrox to 980 g of the above composition.

These blended perfume compositions were compared by a panel of 10 specialists. Six out of the ten panelists reported that the blended perfume composition comprising the compound of this invention had better fragrance and duration.

EXAMPLE 6

The following substances were blended as constituent components of a blended perfume composition of an orchid note.

| Component | Parts by weight |
|---|---|
| Cyclopentadecanolide | 50 |
| Coumarin | 10 |
| Heliotropin | 30 |
| Ylang-Ylang oil | 80 |
| Methyl ionone | 100 |
| Neroli oil | 30 |
| Hydroxycitronellal | 50 |
| Linalool | 70 |
| iso-Butyl salicylate | 110 |
| 5-Cyclohexadecenone | 20 |
| Amyl salicylate | 140 |
| Oak moss absolute | 3 |
| Vanillin | 7 |
| Phenylacetaldehyde | 20 |
| Phenyl ethyl alcohol | 200 |
| Benzyl acetate | 60 |
| | 1000 |

By adding 90 g of (±)-ambrox to 910 g of the above composition, a novel blended perfume composition which was of a fresh and more natural orchid note and had an excellent duration was prepared.

A blended perfume composition was prepared by

adding 90 g of (-)-ambrox instead of (±)-ambrox to 910 g of the above composition.

The above blended compositions were compared by a panel of 10 specialists. Eight out of the ten panelists reported that the blended perfume composition comprising the compound of this invention was longer lasting and had a more fresh and natural ordchid note.

What is claimed is:

1. (±)-3a,6,6,9a-Tetramethyldodecahydronaphtho-(2,1,b]furan of the following formula (A):

(±) 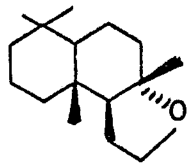 ..... (A)

2. A process for producing (±)-3a, 6, 9a-tetramethyl-dodecahydronaphtho(2,1,b)furan of the following formula (A)

(±) 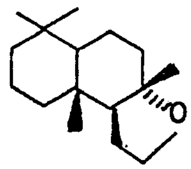 ..... (A)

which comprises

(i) reacting a (±)-trans-beta-monocyclohomo-farnesylic acid or an ester thereof represented by the following formula (1)

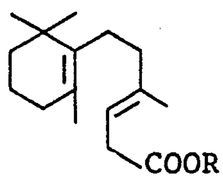 ..... (1)

wherein R represents a hydrogen atom or a methyl or ethyl group,

with trifluoroacetic acid to form decahydro-3a,6,6,9a-tetramethyl-(3aα,5aβ,9aα,9bβ)-(±)-naphtho[2,1-b]furan-2(1H)one of the following formula (2)

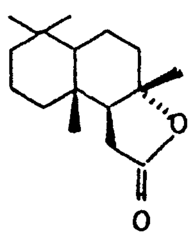 ..... (2)

(ii) reducing the compound of formula (2) with bis-(2-methoxyethoxy)-aluminum sodium hydride

$[NaAlH_2(OCH_2CH_2OCH_3)_2]$ to form (±)-2,5,5,8a-tetramethyl-1-(2-hydroxyethyl)-2-hydroxydecalin of the following formula (3)

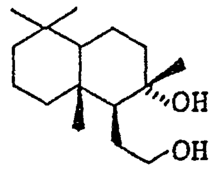

..... (3)

and

(iii) cyclizing the compound of formula (3) by contacting it with p-toluenesulfonyl chloride in the presence of a base to form the compound of formula (A).

3. A perfuming or flavoring composition comprising a conventional perfuming or flavoring agent or carrier and, as a perfuming or flavoring modifier thereof, a modifying amount of (±)-3a, 6, 6, 9a-tetramethyldodecahydronaphtho-(2,1,b)furan of the following formula (A):

(±) 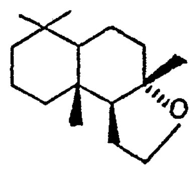

..... (A)

4. A composition according to claim 3 wherein the compound of formula (A) is present in an amount of about 0.001 to about 50% by weight based on the weight of the composition.

5. Use of the compound of formula (A) set forth in claim 1 as a perfuming or flavoring modifier.